# EUROPEAN PATENT APPLICATION

(11) **EP 3 653 196 A1**
(43) Date of publication of application: **20.05.2020**
(21) Application number: 18832087.3
(22) Date of filing: 21.05.2018
(51) Int. Cl.: A61K 8/06, A61K 8/19, A61K 8/72, A61K 8/73, A61Q 19/00

(54) **COSMETIC MASK FOR APPLICATION TO THE SKIN, AND METHOD FOR APPLYING SAME**

(30) Priority: 11.07.2017 RU 2017124799
(71) Applicant: Limited Liability Company "Blom Rus" ("Blom Rus" LLC), Moscow 115191 (RU)
(72) Inventor: ZVEZDIN, Vasiliy Nikolaevich, g. Perm 614051 (RU); AKAFIEVA, Tatyana Igorevna, g. Perm 614038 (RU); KASATKIN, Ivan Arkadievich, g. Perm 614038 (RU)
(74) Representative: Berggren Oy, Tampere
(86) International application number: PCT/RU2018/000314
(87) International publication number: WO 2019/013670

(57) **Abstract**

The inventions relate to cosmetology and are intended to increase a cosmetic effect. A mask comprises a cream-type cosmetic agent and an additional reagent, which are isolated from one another. The agent contains a water-soluble biocompatible polymer which has a mass in the range of 50,000-1,000,000 Da and is capable of entering into a cross-polymerisation reaction. The reagent consists of a substance-electrolyte solution containing multicharged ions. The mass ratio of polymer and substance-electrolyte is from 10:1 to 1:1 respectively. A method involves separately applying to the skin the cream-type agent and then the additional reagent on top.

## Description

### FIELD OF TECHNOLOGY

The invention relates to cosmetology and, in particular, to skin care products and the method of applying them to the skin, mainly on the face, neck, décolleté, hands, intended to cleanse, moisturize, nourish the skin, as well as to eliminate exfoliation and to rejuvenate skin.

### PRIOR ART

In cosmetic practice, the creamy masks are widely used, which, after application, can form a tight-fitting layer on the skin that slightly tightens the skin when it dries.

The use of masks for facial skin is widely known (for example, Brief Encyclopedia of Household, M., Publishing House "S.E.", 1984, p. 253). Almost every mask consists of a base and additional nutritional and therapeutic ingredients. The base may be formed by yolk, protein, starch - rice or maize, oatmeal, yeast, vegetable fats, etc.

Creamy masks represent the most numerous group among the known cosmetic masks with their base rich in fatty nutrients (for example, the author's certificate of the USSR No. 1803097). The fat base is well compatible with a wide range of biologically active substances of plant and animal origin.

It is known that substances of mineral origin can be used as components of masks or bases for them. Such masks, as a rule, are used to reduce the amount of oil on the skin and to clean the skin. This is true, in particular, for a mask base made from white clay mixed with other powdery substances. For some skin types, it is recommended to use the following composition as a base: white clay 3 parts, rice or maize starch 1 part, zinc oxide 1/2 part, magnesia 2 parts, purified talc or tooth powder 2 parts, alum powder 1/5 part ( S.N. Lensina. Cosmetics, age and season. Tallinn "Valgus", 1986, p. 121).

There is a known mask for facial skin care (RF Patent No. 2030911), which helps to cleanse the skin, eliminate exfoliation of the surface comedones and reduces oilness. This mask contains kaolin, talc, bentonite clay or tiha-askanae, as well as ground fruit bones or mineral eggshell fortifier, carbamide, lecithin, chamomile oil extract and other components of plant origin.

However, all of the above analogues relate to single-phase masks, and the main cosmetic effect is achieved through mechanical tightening of the skin. Moreover, the penetration of active cosmetic components through the skin is extremely complicated by the high viscosity of the applied single-phase hardening mask.

Also, there are known cosmetic masks with an emulsion base of water/oil type (a creamy state is maintained due to the presence and ratio of fats and water), which are based on vegetable oils and emulsifiers. Emulsion cream is a cream containing a dispersed system consisting of two immiscible liquids, one of which (dispersed phase) is distributed in the other (dispersion medium). Emulsifiers are used to form emulsions - substances that contribute to the formation of an emulsion from two of these fluids (http://refleader.ru/jgebewjgeotraty.html).

Also there are known cosmetic gel - masks, with the base formed by a structured liquid phase with high water content. Cosmetic masks in the form of a gel are convenient to use, they fit well on the skin and are held on it, and have a high moisturizing effect. However, the assortment of cosmetic gel-masks is very limited, which can be explained by significant difficulties associated with obtaining stable gel structures, especially in the presence of complex biologically active complexes.

For example, there is known cosmetic gel- mask (RF Patent Nº 2054285) containing gelatin, glycerin, tincture of propolis, honey, salicylic acid, sodium chloride, and furacilin and water.

The required cosmetic effect is achieved by exposing the facial skin to electric current or massage through a gel- mask applied to it. Preliminary layer of the nourishing cream is applied to the skin. All this complicates the application of the known gel- mask.

There is known cosmetic mask in the form of an aqueous gel (US Patent No. 5194253), which contains 0.1-1% alkaline or ammonium salt of hyaluronic acid as a biologically active additive and 1-3% of the mineral or organic salt of deoxyribonucleic acid (DNA), as well as a polymer thickener, water and preservative.

The cosmetic effect of using the said gel mask is achieved by implementing a rather complicated cosmetic procedure: applying gel-mask as the first layer on the skin of the face, coating gel layer with gauze, performing massage, applying a second layer of the gel- mask.

Also, from the RF Patent No. 2276998 it is known about hydrogel compositions which (a) have a continuous hydrophobic phase and a discrete hydrophilic phase, (b) have a discrete hydrophilic phase and a continuous hydrophilic phase, or (c) completely consist of a continuous hydrophilic phase. The hydrophobic phase, if present, consists of a hydrophobic polymer, especially a hydrophobic pressure sensitive adhesive (PSA), a plasticizing elastomer, a tackifying resin and an optional antioxidant. The discrete hydrophilic phase, if present, consists of a crosslinked hydrophilic polymer, for example, a crosslinked cellulosic polymer, such as crosslinked sodium carboxymethyl cellulose. For hydrogel compositions containing a continuous hydrophilic phase, the phase components include a cellulose ester composition, or an acrylate polymer, or a copolymer, and a mixture of a hydrophilic polymer and a complementary oligomer capable of forming hydrogen bonds with the polymer. Films obtained from hydrogel compositions containing or wholly consisting of the aforementioned continuous hydrophilic phase can be transparent and can be obtained by extrusion of a melt or casting from a solution.

However, these known compositions have the following disadvantages: - in the proposed formulation of hydrogel compositions, it is suggested to use plasticizers of synthetic origin in sufficiently high concentrations of 25-45 wt.%, which, on the one hand, can potentially cause allergic reactions, on the other hand, leads to an increase in the cost of the product due to the use of plasticizers with complex compositions and structure .

On the contrary the advantage of the proposed cosmetic mask is a use as the plasticizing component of the ionic cross-linking agent that is an aqueous solution of an electrolyte substance, containing multiply charged ions, which has more simple composition and structure (and the concentration of the electrolyte substance is not more than 15 wt%, and mainly 1-5 wt.%), moreover, not directly contained in the component applied to the skin. As a result, allergic reactions will be excluded and, in addition, the cost of the proposed mask is lower than claimed in the aforementioned patent.

In the invention proposed in the known said patent No. 2276998, the layers of the applied mask are not separated, thus, there are no useful cosmetic effects (preservation of biologically active components, increased penetration of the components into the skin, "spa effect", etc.) because hydrogel compositions have a fairly high viscosity.

From the patent of the Russian Federation No. 2383341 it is known about a method of skin rejuvenation, according to which skin treatment is carried out in two stages by the sequential application of hyaluronic acid by applying the first and second cosmetic compositions to the skin, using the first cosmetic composition containing hyaluronic acid in the form of ultrafine particles. At the first stage, hyaluronic acid is applied as a part of the first cosmetic composition in the form of a gel and/or serum, at the second stage, the second cosmetic composition is applied using application tools.

The disadvantages of this method are the problematic use of oils and/or oil-soluble cosmetic components, which significantly limits the range of ingredients, as well as the low stability of the cosmetic product, due to the use of a colloidal solution of ultrafine particles of hyaluronic acid.

Closest to the proposed invention and the composition of the mask and the method of its application is the technical solution described in RF Patent No. 2138244 "Cosmetic and/or dermatological product for topical application to the skin and the method of cosmetic and/or dermatological treatment of the skin." A known product for topical application, capable of releasing a cosmetically and/or dermatologically active substance on the skin, contains a lipase as an enzyme and at least one ester, an active substance precursor comprising an ester function with a linear or branched, saturated or unsaturated chain with 2-25 carbon atoms.

According to a preferred embodiment of the invention, the precursor and lipase are packaged so as not to come into contact with each other until applied to the skin. For example, it is proposed to place the compositions in two compartments that are connected through a common channel, from where they can come out, mixing, before applying to the skin. Such a two-compartment package is described, for example, in French patents A-204559, A-2105332, A-2258319, A-2293375, A-2586913 or A-2643615.

The said patent also describes a method for applying the product to the skin, according to which the precursor and lipase, packaged individually, are applied to the skin sequentially or with an interval in time.

The disadvantages of this known product and method of its application are the following:
- the use of enzymes in the composition of the product complicates the conditions of storage, transportation and use of a cosmetic mask;
- the actual absence of a barrier preventing the oxidation of the components of a known product with atmospheric oxygen can lead to the destruction of biologically active components of the mask due to exposure to ultraviolet radiation;
- the nature and sequence of procedures for applying the product to the skin do not allow the claimed cosmetic effects stated in the invention to be applied: preservation of biologically active components, increased permeability of the components to the skin, "lifting effect".

### BRIEF SUMMARY OF THE INVENTION

Single technical result of the group of inventions is the improvement of the cosmetic effect of superimposed proposed skin mask due to protection of the active ingredients from oxidation; protection of the mask from moisture loss, which means increased hydration of the skin; while creating a lifting effect and increasing the efficiency of penetration of active components into the epidermis.

The said technical result is achieved by the use of the proposed cosmetic mask for application to the skin, including a creamy cosmetic product with a fat, emulsion, or gel base and an additional reagent isolated from each other.

The novelty is that the specified creamy cosmetic product additionally contains in an amount of 0.01-10.0 wt.% water-soluble biocompatible polymer with a weight in the range of 50,000 - 1,000,000 Da, capable of entering into a cross-polymerization reaction and selected from the group including: polyvinyl alcohol, polyvinylpyrrolidone, water-soluble modifications of polysaccharides, water-soluble copolymers of polysaccharides and mixtures thereof; and as an additional reagent, the mask contains a 0.5-15.0% aqueous solution of an electrolyte substance containing multiply charged ions, while the weight ratio of the indicated water-soluble biocompatible polymer in a creamy cosmetic product to an electrolyte substance in an aqueous solution is from 10 : 1 to 1: 1, respectively.

Soluble biocompatible polymers include in their structure free, non-chemical monomers, groups selected from the series including: hydroxyl group, carbonyl group, carboxyl group; a carboxyl group that has formed salt-like compounds with singly charged cations, or a combination of the above groups.

As soluble polysaccharide modifications polymer is used, selected from the group consisting of, for example: hydroxyethyl starch, chitosan, carrageenan; as water-soluble copolymers of polysaccharides polymer is used, selected from the group consisting of, for example, xanthan gum, sodium alginate; and mixtures thereof are used as polymers, selected from the group consisting of, for example, agar - agar.

As a mixture of these polymers in a creamy cosmetic product, a two-component mixture of these polymers is used in the range of weight ratios from 1: 100 to 1: 1.

The creamy cosmetic product in its base contains oils: basic and/or essential; and/or cosmetic emulsifiers; and/or emollients; and/or active components; and water.

As basic oils, the creamy cosmetic product in its base contains ExtraVirgin olive oil, and/or grape seed oil, and/or Shea butter, and/or Babassu oil, and/or sesame oil.

As essential oils, the creamy cosmetic product in its base contains bergamot essential oil and/or vanilla essential oil and/or tea tree essential oil and/or ylang - ylang essential oil and/or sandalwood essential oil and/or patchouli essential oil and/or lavender essential oil.

As cosmetic emulsifiers, the creamy cosmetic product in its base contains cetyl alcohol and/or cetearyl alcohol and/or lecithin and/or beeswax and/or hydroxyethyl cellulose and/or polyethylene glycol and/or polyvinyl alcohol.

As emollients, the creamy cosmetic product in its base contains substances selected from the group, including: mineral oils, emollients of natural origin, for example, lanolin, ceresin, low molecular weight silicones.

As active components, the cosmetic product in its base contains substances selected from the group, including: hyaluronic acid, or allantoin, or inulin, or herbal extracts, or urea.

The cosmetic product additionally contains cosmetic components that improve consumer properties, namely: fragrances, and/or pigments, and/or preservatives.

As an electrolyte substance containing multiply charged ions, it contains salts of divalent metals, for example, calcium chloride, or zinc chloride, or zinc sulfate, or magnesium sulfate; or polybasic organic and inorganic acids and/or their salts, for example, boric acid, or citric acid, or sodium citrate.

An additional reagent is an aqueous solution of an electrolyte substance, which contains distilled or double distilled or flower water as water.

As flower water, an aqueous solution of an electrolyte substance contains lavender flower water, or lemon balm flower water, or pink flower water.

The said technical result is also achieved by the proposed method of applying a cosmetic mask to the skin, including applying a creamy cosmetic product with a fat, or emulsion, or gel base on the skin and then applying an additional reagent to it. The novelty is that initially a creamy cosmetic product is applied to the skin which contains an additional water-soluble biocompatible polymer with a weight in the range of 50,000 to 1,000,000 Da, capable of undergoing a cross-polymerization reaction, and selected from the group consisting of: polyvinyl alcohol, polyvinyl pyrrolidone, water-soluble modified polysaccharide, water-soluble copolymers of polysaccharides and mixtures thereof; they are incubated for 5-7 minutes and then an additional reagent is applied on the layer of the said product - 0.5-15.0% aqueous solution of an electrolyte substance containing multiply charged ions, while the weight ratio of the said water-soluble biocompatible polymer in a creamy cosmetic product and the electrolyte substance in the aqueous solution is from 10: 1 to 1: 1, respectively.

An aqueous solution of an electrolyte substance is applied in the form of a spray or aerosol sprayed onto the surface of the applied creamy cosmetic product for no more than 2-3 seconds.

The predefined technical result is achieved due to the following.

Skin is one of the most vulnerable organs in the body. The skin, directly or indirectly, is in constant interaction with external stimuli and is often exposed to and is affected by environmental factors. Indeed, skin can be considered as the first frontier in contact with the outside world. Such constant exposure can lead to unpleasant and/or undesirable physical and visible changes in the skin, in particular, changes in the cosmetic condition of the skin. Despite the fact that such changes may not threaten the health of the individual, they can cause physical discomfort or lead to the formation of visible defects. Since the skin is often visible, changing its appearance can cause psychological stress. Thus, there is a constant need and demand for effective skin care products designed to maintain, restore or improve the condition of the skin and, in particular, to restore the youthful appearance of the skin.

The proposed cosmetic mask consists of two parts: a creamy cosmetic product on a fat, or emulsion, or gel base with the addition of an additional water-soluble biocompatible polymer with a weight in the range of 50,000 - 1,000,000 Da, capable of undergoing cross-linking and undergoing cross-polymerization reaction, which is applied as a layer directly on the skin (hereinafter referred to as component No. 1), as well as an aqueous solution of an electrolyte substance containing multiply charged ions (hereinafter referred to as component No. 2), which are isolated from each other until and application to the skin.

As the said water-soluble biocompatible polymer, it is recommended to use a polymer selected from the group including: polyvinyl alcohol, polyvinyl pyrrolidone, water-soluble modifications of polysaccharides (e.g. hydroxyethyl starch, chitosan, carrageenan and other polymers of this group), water-soluble copolymers of alumina, polysaccharides sodium and other polymers of this group), their mixtures (for example, agar - agar). These polymers in the preferred embodiment have in their structure free hydroxyl groups that are not involved in the chemical bond of the monomers (for example, at least 2); and/or carbonyl groups (e.g., at least 1); and/or carboxyl groups (for example, at least 1) or carboxyl groups that form salt-like compounds with cations (for example, Na +, K +); or a combination of the above groups. These features characterize a group of polymers with a weight in the range of 50,000-1,000,000 Da that are capable of reacting with ionic cross-polymerization, using substances that are prone to dissociation in protogenic polar solvents (for example, water) as a crosslinking agent.

When implementing the claimed method of applying the claimed cosmetic mask, after component No. 1 is applied onto the skin, component No. 2 is applied on top of the applied area (in the preferred embodiment, it is assumed that component No. 2 is applied by spraying with a spray or a soft brush). After that, in the applied component No. 1, two layers can be conditionally distinguished: the outer one is a dense gel layer crosslinked with component No. 2, identical in composition to component No. 1, but different from the latter with a higher viscosity and lower water and fat solubility; and the inner layer is a layer directly adjacent to the skin, but not subjected to ionic cross-linking component No. 1. That is, the actual proposed mask after application becomes two-layer, but there will be no difference in the composition of the layers, because their composition is determined by the composition of the creamy cosmetic product with addition of water-soluble biocompatible polymers.

The concentration of water-soluble biocompatible polymers capable of undergoing cross-linking in component No. 1 is from 0.01 to 10.0 wt. % Various types of water-soluble biocompatible polymers are given in the book Marychev S.N., Kalinin B.A. M25 Polymers in medicine: Textbook/Vladim. State University; Vladimir, 2001.68 p. http://e.lib.vlsu.ru/bitstream/123456789/664/1/MAR.pdf).

The concentration of the ionic cross-linking agent (an electrolyte substance containing multiply charged ions) in component No. 2 is from 0.5 to 15 wt%.

In the formulation of the proposed cosmetic mask, such an aqueous solution of cross-linking agents (component No. 2) is selected that, when added in the claimed amount to the said creamy cosmetic product on a fat, or emulsion, or gel base, plasticizes its outer surface to a gel consistency.

Moreover, due to the claimed weight ratio of a water-soluble polymer in a creamy cosmetic and an electrolyte substance in an aqueous solution (respectively, in the range from 10: 1 to 1: 1), it became possible to reduce the amount of a water-soluble polymer capable of undergoing a cross-linking reaction by more than twice, for example, in comparison with the hydrogel compositions according to the patent of the Russian Federation No. 2276998, which will provide not only lower cost of the finished product, but also eliminate the negative effect on the skin, which will increase the effectiveness of the cosmetic effect.

The increase in the cosmetic effect is due to the fact that when applying the claimed cosmetic mask on the skin, two layers are formed with the following functions:
- the upper gel layer provides protection of the underlying creamy cosmetic product from oxidation, from moisture evaporation, which contributes to intensive skin hydration and increased skin permeability to the active ingredients of component No. 1. Along with this, such a mask with an upper gel layer will provide a modeling lifting effect;
- the lower layer, consisting of a creamy cosmetic product, retains its viscosity and molecular weight of the matrix, which contributes to the deep penetration of active cosmetic substances into the epidermis and dermis.

The mechanism of interaction of component No. 1 and component No. 2 is as follows. Water-soluble biocompatible polymers capable of undergoing a cross-linking (cross-polymerization) reaction, which are part of component No. 1, have in their structure ionogenic groups of atoms associated with singly charged ions (for example, H ⁺, Na ⁺, K ⁺, etc..) In turn, component No. 2 incorporates a solution of a salt, for example, divalent metals, for example, calcium chloride, or zinc chloride, or zinc sulfate, or magnesium sulfate; or polybasic organic/inorganic acids and/or organic/inorganic salts forming multiply charged ions in a solution, for example, boric acid, or citric acid, or sodium citrate.

In the interaction of the above groups of substances, singly charged ions in the ionic water-soluble polymer of component No. 1 are replaced by the same charge multiply charged ions of the electrolyte substance of component No. 2, which leads to the formation of cross-polymer hydrogels, which differ in density, viscosity and resistance compared to the original polymer to the effects of hydrophilic and hydrophobic solvents, oxidizing agents.

Due to the fact that the interaction of the specified polymer and electrolyte occurs at the reagent interface: "component No. 1 - component No. 2" (the use of component No. 2 is preferably in aerosol form for application to component No. 1), the formation of the hydrogel occurs only on the outer surface of the applied plasticizing masks, which makes it essentially biphasic (the upper and lower layers of a creamy cosmetic product, different in viscosity, are in contact with the skin).

### DETAILED DESCRIPTION OF THE INVENTION

In the process of testing the following water-soluble biocompatible polymers were studied, selected from the claimed:

Polyvinylpyrrolidone (from now on - PVP) is a biopolymer from the poly group (N-vinyl lactam), which is part of the blood plasma. Mass up to 360,000 Da. In cosmetics, a mixture of polymers of various molecular weights that have a lifting effect on the skin is used (http://cosmobase.ru/handbook/show/PVP).

Polyvinyl alcohol is a carbochain polymer. The chemical formula of polyvinyl alcohol is (C2H4O)x, where x is responsible for the degree of polymerization. Mass from ∼ 90,000 to ∼ 200,000 Da. Due to its properties, polyvinyl alcohol is used as a modifier and thickener in the composition of polyvinyl acetate adhesives. This substance is used as a stabilizer of emulsion polymerization, as well as a protective colloid in the manufacturing process of polyvinyl acetate dispersions.

Xanthan gum (Xanthan gum) is a branched polysaccharide, a microbial extracellular polysaccharide that is a product of the fermentation of the bacterium Xanthomonas Campestris. Mass ∼ 1 000 000 Da. It is widely used as a gelling agent, thickener and emulsion stabilizer. Xanthan gum gels are resistant to alcohols, surfactants, acids and alkalis. Also, xanthan gum solutions have the property of thixotropy, which manifests itself in a wide pH range (2 - 12).

Chitosan is a naturally occurring linear polysaccharide consisting of acylated and non-acylated D-glucosamine units. Mass from 50,000 to 1,000,000 Da. Due to the presence of a large number of amino groups, it is able to acquire a partially positive charge in solutions, to form a dense network of hydrogen bonds, due to which chitosan has exceptional sorbing properties of both organic substances and metal ions. It is used in cosmetics (as part of gels, shampoos, creams, etc.), in medicine (accelerating wound healing systems), in water treatment systems, in the textile and food industries.

Starch is a natural polymer composed of α-glucose units. Mass 60,000 to > 600,000 Da. It is soluble in hot water, forming three-dimensional gel structures due to the partial exit of small amylase molecules from the crystalline starch domains. It is used in the manufacture of paper, textile, pharmaceutical and food industries as a thickener, sizing, flocculant, filler, adhesive and viscosity modifier. Due to its wide availability and residually high reactivity, starch is used as a raw material for the synthesis of modified starches for specific technical needs.

However, it should be noted that the scope of the water soluble biocompatible polymers claimed in the present invention is not limited thereto. They are given only as separate examples to illustrate the invention.

The creamy cosmetic product that is part of the proposed mask contains a water-soluble biocompatible polymer capable of forming elastic water-fat-insoluble polymer gels by ion cross-polymerization. And along with this, the specified cosmetic product may include any traditional cosmetic ingredients, for example, oils: base and/or essential; and/or cosmetic emulsifiers; and/or emollients; and/or active ingredients, and water. It may also include, for example, surfactants (hereinafter referred to as surfactants), for example, polyoxyethylene monolaurate, water and fat soluble extracts, pigments, preservatives, flavorings, etc. That is, in fact, you can use any ready-made cosmetic products of any type in the proposed cosmetic mask, depending on the desired cosmetic effect, add the mentioned polymer to them in the claimed ratio, add (in isolation before the procedure for applying to the skin) an aqueous solution of an electrolyte substance, fragments of molecules which act as nodes of ionic cross-polymerization of these polymers, and get the claimed cosmetic mask. Testing was performed with various types of creamy cosmetic products.

It should be clarified that traditionally a creamy cosmetic product is a fat-based one, in the form of an emulsion, in the form of a gel (http://allrefs.net/c12/3szr8/p6/; http://www.cosmetika-gloris.ru/vse-o-kosmetike/sostav-kosmetiki).

As the fatty substances used in such product, there can be mentioned, for example, base oils: ExtraVirgin olive oil and/or grape seed oil and/or Shea butter and/or Babassu oil and/or sesame oil; essential oils: bergamot essential oil and/or vanilla essential oil and/or tea tree essential oil and/or ylang-ylang essential oil and/or sandalwood essential oil and/or patchouli essential oil and/or lavender essential oil.

Iti is possible to use, for example, mineral oils (petroleum jelly, mineral oil), vegetable oils and their hydrogenated derivatives, animal oils, synthetic oils, silicone oils (dimethicone, cyclomethicone) and fluorinated oils. Other fatty substances include fatty alcohols, fatty acids and waxes.

The composition of the specified cosmetic products may also include, for example, aqueous, alcoholic or aqueous-alcoholic solutions, hydrophilic or lipophilic gels, and may be in the form of microemulsions, oil-in-water or water-in-oil emulsions or water-in-oil- in-water or oil-in-water-in-oil, having the appearance of a cream or gel.

As it is well known, the creamy cosmetic component of the claimed mask may contain, for example, hydrophilic or lipophilic gelling agents, and/or surface-active agents, and/or hydrophilic or lipophilic active substances, and/or preservatives, and/or antioxidants and/or solvents and/or aromatic substances and/or fillers, filters, and/or colorants.

The amounts of various constituents in such products do not exceed the concentrations corresponding to the classically used amounts in this field, for example, determined in accordance with GOST 31460-2012.

In laboratory conditions, a series of experiments were conducted to produce the claimed mask applied to the skin by the proposed method.

The examples shown in Table 1 are given as an illustration to better understand the invention, but the scope of the rights of the present invention is not limited to them.

To prove that the composition of the proposed cosmetic mask as a creamy cosmetic product (component No. 1) products of various cosmetic types may be used, providing consumer properties of creams (nourishing creams, creams for smoothing the skin, moisturizing, emulsion creams, etc.) d.), so such various ingredients were used in the tests. The amount of ingredients is given in weight percent. Component No. 2 is an aqueous solution of an electrolyte substance containing multiply charged ions.

An example of the preparation of the claimed mask (for example 1 from Table 1).
Component No. 1: 18.8 g of cocoa butter, 6.3 g of ceresin, 5 g of cetyl alcohol and 2.5 g of calendula oil extract were mixed in a thermostatically controlled container, the container was heated to a temperature of + 65 °C and mixed using immersion mixers for 5 minutes at a speed of 250 rpm until complete dissolution of the components of the mixture and its homogenization. Next, 59.2 g of hot (+70 °C) water was poured into the mixture heated to +65 °C, after which the heating was turned off and the resulting mixture was stirred for 15 minutes at a rotation speed of 500 rpm. Next, 5.3 g of chitosan, 1 g of polysorbate-80, 0.76 g of starch, 0.5 g of methylisothiazolinone, 0.2 g of geraniol, 0.2 g of rosehip essential oil and 0.2 g of limonene were added to the mixture, cooled to room temperature, after which the mixture was mixed for 25 minutes at a speed of a submersible mixer of 500 rpm. As a result, component No. 1 was obtained (Example 1 of Table 1) with the following ratio of ingredients, wt.%: Cocoa butter - 18.8%; Rosehip essential oil - 0.2%; Cetyl alcohol - 5%; Ceresin - 6.3%; Calendula extract - 2.5%; A mixture of chitosan and starch (7: 1) - 6.1%; other cosmetic substances (polysorbate-80, methylisothiazolinone, geraniol, limonene) - 1.9%; water 59.2%.
Component No. 2: 4 grams of magnesium sulfate and 2 g of citric acid were added to the polyethylene container, after which 94 g of warm (∼ 50 °C) melissa flower water was added. The mixture was stirred with an immersion mixer for 10 minutes at a stirrer speed of 250 rpm. As a result, component No. 2 was obtained with the following content of ingredients, wt.%: Melissa flower water - 94, Citric acid - 2, Magnesium sulfate - 4.

Masks with other ingredients were prepared in a similar way.

All of these cosmetic masks were applied to the skin of the face by the proposed method, i.e. first, component No. 1 was applied to the skin, exposure was performed, and then component No. 2 was applied on top of this component, and exposure was again performed.

During the tests, 15 different masks were tested. At the same time, creamy cosmetics (component No. 1) were used based on base oils and/or essential oils and/or cosmetic emulsifiers and/or emollients and/or active ingredients and water. Component No. 1 also contained water-soluble biocompatible polymers capable of undergoing cross-linking in an amount of 0.01-10.0 wt.%. Various bases were also tested at the same time, namely, ExtraVirgin olive oil, and/or grape seed oil, and/or sesame oil.

As the essential oils, the creamy base consisted of bergamot essential oil and/or vanilla essential oil and/or tea tree essential oil and/or ylang - ylang essential oil and/or sandalwood essential oil and/or patchouli essential oil , and/or lavender essential oil.

As cosmetic emulsifiers, the cosmetic base contained cetyl alcohol and/or cetearyl alcohol and/or lecithin and/or beeswax and/or hydroxyethyl cellulose and/or polyethylene glycol and/or polyvinyl alcohol.

As emollients, the cosmetic product in the base contained capric/capric triglycerides, and/or mineral oils.

And as the active components for the preparation of the said product, hyaluronic acid, or allantoin, or inulin, or plant extracts were used as the base.

As the water there was used distilled or floral water.

Also, in a number of cosmetics, other cosmetic ingredients were additionally introduced: aromatic substances, pigments, preservatives and auxiliary cosmetic substances.

As an aqueous solution of an electrolyte substance containing multiply charged ions (component No. 2), a 0.5-15% solution (solvent: distilled or double distilled or flower water) was used, in which divalent salts were used as an electrolyte substance metals, for example, calcium chloride, or zinc chloride, or zinc sulfate, or magnesium sulfate; or polybasic organic and inorganic acids and/or their salts, for example, boric acid, or citric acid, or sodium citrate.

Moreover, in thirteen experiments, the ratio of water-soluble biocompatible polymers and electrolyte was taken in various ratios of the claimed range from 10: 1 to 1: 1, respectively; in two experiments (Nos. 8 and 12) - 1:10 and 13: 1, respectively (to prove the correctness of the declared quantitative limits of the ratios specified in the claims).

The recipes of the specific investigated examples of cosmetic masks: component No. 1 and component No. 2 are shown in Table 1.

It should be noted that in examples 1-5, the time between application of component No. 1 and component No. 2 was 5 minutes; in examples 6-8 - 7 minutes; in the remaining examples, 6 minutes

The time of face exposure to the claimed mask (from the moment of applying the creamy cosmetic product) for examples 1, 4-6 was 10 minutes; for examples 7-9 - 12 minutes; for other examples - 15 minutes.

In examples 1-9, 14, component No. 2 was applied in the form of a spray, in the remaining examples with a soft brush.

During the experiments, the degree of cosmetic effect of the applied mask was determined in accordance with the dermatological protocol of the European Association of Dermatologists (Jain R., Huang P. A new tool to improve delivery of patient-engaged care and satisfaction in facial treatments: the Aesthetic Global Ranking Scale // Journal of Cosmetic Dermatology, No. 1, p. 1-12.

The study involved 150 volunteer patients, women aged 37 to 45 years. An expert assessment was performed by 10 expert cosmetologists. Changes were evaluated on a rank 4-point scale, where one point is equal to 30% of the degree of change of the sign. The following parameters were selected as criteria: increase in elasticity, decrease in dehydration, decrease in volume loss. Assessment was performed before the start of the course and after 4 procedures for applying the claimed mask. Masks were applied no more than once every 48 hours. Masks were applied by the proposed method in an even layer on the face, avoiding the periorbital zone.

According to the results of the study, it was found that in examples 1, 2, 4, 7, 9-13, 15, component No. 1 had a creamy structure; in other examples, it was gel-like.

In examples 1, 3, 4, 6, 7, 9, 10, 13-15, when applying component No. 1, the mask was applied as an even layer, after applying component No. 2, the upper part of the mask was compacted. After the exposure time on the face, the mask was removed easily without injuring or drying the skin. These examples correspond to the claimed formulation of the mask and the proposed method of its application.

When using masks according to the specified examples 1, 3, 4, 6, 7, 9, 10, 13-15, intensive skin hydration was observed in 100%, elasticity increased in 65-70% of cases.

In examples 2, 5, when applying component No. 1, the masks were applied in an even layer (in these examples, component No. 1 contains a water-soluble biocompatible polymer above the claimed amount), but when component No. 2 was added during compaction of the upper surface of component No. 1, lumps formed, layer became uneven. These masks could only be removed with water, by rinsing. At the same time, dry skin was observed at the sites of lump formation; skin irritation associated with mechanical removal of masks was observed in 10-12% of cases.

In examples 8, 11, when applying component No. 1, the masks also were applied in an even layer (in these examples, component No. 1 contains a water-soluble biocompatible polymer lower than claimed), when component No. 2 was added, the upper surface of component No. 1 did not seal. These masks could only be removed with water, by rinsing. When using the masks from examples 8, 11, skin hydration was observed only in 30%, an increase in elasticity was observed in 10%.

In example 12, when applying component No. 1, the mask was applied in an even layer (component No. 1 contains a water-soluble biocompatible polymer in the claimed formulation), when component No. 2 was added (component No. 2 contains a solution of an electrolyte substance below the claimed), compaction of the upper surface of component No. 1 did not occur. This mask could only be removed with water, by rinsing. When applying the mask of example 12, skin hydration was observed in 40%, an increase in elasticity was observed in 10%.

On the basis of the formulation specified in examples 1, 3, 6, the primary combination of component No. 1 and component No. 2 was carried out directly during mask preparation, in violation of the previously specified application algorithm. When preparing it, after 30 seconds a gel-like structure formed with compacted gel lumps, which was almost impossible to apply to the skin. After 24 hours, the structure divided into layers with an effusion of the oil phase. The use of such a mask was not possible, due to the loss of consumer qualities and the difficulty of application.

Tests were also carried out with the claimed masks with other polymers, such as: hydroxyethyl starch, carrageenan, sodium alginate, agar - agar. The results were similar to the positive examples shown in Table 1.

Thus, the proposed cosmetic mask applied to the skin using the claimed method has the following advantages over the known products:
- provision of a modeling lifting effect without the use of additional cosmetics;
- Prevention of moisture loss and ensuring effective hydration of the skin in 100% of experiments;
- increased efficiency of penetration into the epidermis of the active components located in the inner layer of the mask;
- protection of the active components of the mask from oxidation during interaction with atmospheric oxygen.

All of the above allows increasing the cosmetic effect of the proposed mask, applied to the skin using the claimed method.

**Table 1**

| NºExample | Creamy cosmetic product (component No. 1), mass% | | | | | | | | Component Nº2 | Texture |
|---|---|---|---|---|---|---|---|---|---|---|
| | Oil | | cosmetic emulsifier | emollient | active component | water-soluble biocompatible polymer | Other cosmetic substances | water | | |
| | basic | essential | | | | | | | | |
| 1 | 2 | 3 | 4 | 5 | 6 | 7 | 8 | 9 | 10 | |
| 1 | Cocoa butter - 18.8% | Rosehip Essential Oil - 0.2% | Cetyl alcohol-5% | Ceresin - 6.3% | Calendula extract - 2.5% | A mixture of chitosan and starch (7:1)-6.1% | polysorbate-80, methylisothiazol inone, geraniol, limonene - 1.9% | 59.2% | melissa flower water - 94%, Citric acid - 2% Magnesium sulfate - 4% | cream-type |
| 2 | Shea butter - 11% | patchouli essential oil-0.1% | Emulsion wax - 3% | Castor oil - 3.7% | Inulin - 3% | Polyvinyl alcohol - 18.5% | PEG - 600, sodium benzoate, potassium sorbate - 2.2% | 58.5% | Lavender flower water - 92%; sodium borate - 5% + boric acid - 3% | cream -type |
| 3 | - | Sandalwo od essential oil - 0.1% - | - | Isopropyl palmitate - 8.1% | Panthenol - 4% | Polyvinylpyrrolidone / starch (1: 3) - 4.9% | Alpha - lipoic acid, citrus terpenes, eucalyptolum - 3.4% | 79.5% | Distilled water - 97% sodium citrate - 2%, boric acid - 1% | gel-type |
| 4 | Olive oil Extra virgin - 25.3% | - | Cetearyl alcohol - 5% | - | Allantoin - 3.2% | Xanthan gum - 0.2% | Polysorbate - 20, methylisothiazolinone, geraniol, limonene - 2.5% | 63.8% | Distilled water - 99.4% sodium borate - 0.1% citric acid - 0.1% | cream -type |
| 5 | - | Lavender essential oil - 0.2% | - | Isopropyl myristate - 11.4% | Urea - 6% | Starch, polyvinylpyrrolidone (10: 1) - 21.5% | Polysorbate - 80, phenoxyethanol, methylisothiazol inone, limonene -2.3% | 58.6% | Double distilled water - 94%, citric acid - 3%, boric acid - 1% | gel-type |
| 6 | - | - | - | - | Urea + hyaluronic acid (20: 1) - 11.6% | Starch / Xanthan Gum (92:1) - 4.7% | PEG - 600, microcrystalline cellulose, methylparaben, geraniol, limonene - 6.2% | 77.5% | Flower pink water - 98.8 Sodium citrate - 1.0%, citric acid - 0.2% | gel-type |
| 7 | Sesame oil - 16.8% | - | - | Capric / capric triglycerides - 10% | sage extract - 2.7% | Polyvinyl alcohol polyvinylpyrrolidone (1: 2) - 1.5% | Azelaic acid, phenoxyethanol -2% | 67% | Melissa Flower Water - 99.85% sodium borate-0.15% | cream-type |
| 8 | - | Bergamot essential oil - 0.2 % | Polysorba te - 20 - 10.5% | Cyclodimethicone - 7% | Coenzyme Q10 - 3.5% | Chitosan / polyvinyl alcohol (1: 5) - 0.007% | Titanium Dioxide, methylisothiazolinone - 4.9% | 73.893 % | Double-distilled water - 95%, sodium borate - 0.5%, zinc sulfate -0.5% | gel-type |
| 9 | Apricotoil - 21% | Essential oil ylang - ylang - 0.1% | Wax tree Nimes - 9% | - | Inulin - 1. 1% | Polyvinylpyrrolidone - 0.01% | Glycerin monostearate, methylisothiazolinone - 3% | 65.79% | Floral rose water - 99.99% boric acid - 0.01% | cream-type, a large excess of spray |
| 10 | Macadamia oil - 30% | - | Cetearyl alcohol - 8.2% | - | Collagen - 3.3% | Chitosan - 2.7% | Phenoxyethanol, methylparaben - 1.5% | 54.3% | Lavender flower water - 98.2 Magnesium Sulfate - 1.8% | cream-type |
| 11 | Palm kernel oil-22.6% | - | Emulsion wax-3.2% | Lanolin-5.2% | - | Starch / polyvinylpyrrolidone (6: 1) - 0.005% | Zinc oxide, methylisothiazolinone, limonene -2.6% | 66.395 % | Distilled Water - 90 Sodium citrate - 9%, boric acid - 1% | cream-type |
| 12 | Babassu Oil - 15% | Cinnamon Essential Oil - 0.3% | Cetearyl alcohol - 4.1% | - | Chamomile extract - 4.8% | Xanthan gum / polyvinyl alcohol (6: 1) -7.9% | Carboxymethyl cellulose, potassium sorbate, sodium benzoate - 4.1 % | 63.8% | Flower pink water - 99.7% citric acid - 0.6%, | cream -type, little sprey |
| 13 | Palm Oil - 20.1% | - | Nimes Wax - 4.9% | Capric / Capric triglyceride s - 3.8% | Squalane - 2.5% | Polyvinyl alcohol / starch (1: 2) - 3.8% | Phenoxyethanol, methylisothiazol inone, geraniol, limonene - 1.9% | 63% | Double-distilled water - 99.62%, sodium borate - 0.38% | cream-type |
| 14 | | Verbena essential oil-0.1% | Polysorba te - 20 - 3.3% | Ceresin - 4.9% | Allantoin - 3.3% | Triethyl citrate, sodium benzoate, potassium sorbate, lavender -4.2% | Triethyl citrate, sodium benzoate, potassium sorbate, lavender - 4. 2% | 82.5% | Flower pink water - 98.3%, magnesium sulfate - 0.5%, boric acid - 1.2% | gel-type |
| 15 | Jojoba oil - 23.8% | Lemongra ss essential oil - 0.2% | Emulsion wax-4% | Isopropyl palmitate - 2.4% | Q10 + inlin (1: 4) - 4.1% | Starch / chitosan (19: 1) - 4% | Polysorbate - 20, Eucalyptol, lavender, geraniol - 3.5% | 58% | Melissa flower water - 97.5% citric acid - 2.5% | cream-type |

## Claims

1. A cosmetic mask for applying to a skin, comprising a creamy cosmetic product on a fat, emulsion, or gel base and an additional reagent isolated from each other, **characterized in that** the creamy cosmetic product additionally contains in an amount of 0.01-10.0 wt.% a water-soluble biocompatible polymer with a mass in the range 50,000 - 1,000,000 Da, capable of undergoing a cross-polymerization reaction and selected from the group consisting of: polyvinyl alcohol, polyvinyl pyrrolidone, water-soluble modifications of polysaccharides, water-soluble copolymers of polysaccharides and mixtures thereof; and the additional reagent of the cosmetic mask contains a 0.5-15.0% aqueous solution of an electrolyte substance containing multiply charged ions, while weight ratio of the water-soluble biocompatible polymer to the electrolyte substance in an aqueous solution is from 10:1 to 1:1, respectively.

2. The mask according to claim 1, **characterized in that** the water-soluble biocompatible polymer includes in its structure free groups, which do not form chemical bonds with monomers, selected from the following: hydroxyl group, carbonyl group, carboxyl group; a carboxyl group that has formed salt-like compounds with singly charged cations, or a combination of the above groups.

3. The mask according to claim 1, **characterized in that** the water-soluble modifications of the polysaccharides include a polymer selected from the following: hydroxyethyl starch, chitosan, carrageenan; the water-soluble copolymers of polysaccharides include a polymer selected from the following: xanthan gum, sodium alginate; and the mixtures thereof include polymers selected from following: agar - agar.

4. The mask according to claim 1 or claim 3, **characterized in that** as the mixture of the polymers in the creamy cosmetic product a two-component mixture of these polymers is used in the range of weight ratios from 1:100 to 1:1.

5. The mask according to claim 1, **characterized in that** the creamy cosmetic product in its base contains oils: basic and/or essential; and/or cosmetic emulsifiers; and/or emollients; and/or active components; and water.

6. The mask according to claim 1 or claim 5, **characterized in that** as basic oils a creamy cosmetic contains in its base an ExtraVirgin olive oil and/or grape seed oil and/or Shea butter and/or Babassu oil and/or Sesame oil.

7. The mask according to claim 1 or claim 5, **characterized in that** as essential oils the creamy cosmetic product contains in its base bergamot essential oil and/or vanilla essential oil and/or tea tree essential oil and/or ylang-ylang essential oil, and/or sandalwood essential oil, and/or patchouli essential oil, and/or lavender essential oil.

8. The mask according to claim 1 or claim 5, **characterized in that**, as cosmetic emulsifiers, the creamy cosmetic product contains in its base cetyl alcohol and/or cetearyl alcohol and/or lecithin and/or beeswax and/or hydroxyethyl cellulose, and/or polyethylene glycol and/or polyvinyl alcohol.

9. The mask according to claim 1 or claim 5, **characterized in that**, as emollients, the creamy cosmetic product in its base contains substances selected from the group, including: mineral oils, emollients of natural origin, for example, lanolin, ceresine, low molecular weight silicones.

10. The mask according to claim 1 or claim 5, **characterized in that**, as the active components, the cosmetic product in its base contains substances selected from the group, including: hyaluronic acid, or allantoin, or inulin, or herbal extracts, or urea.

11. The mask according to claim 1, **characterized in that** the cosmetic product additionally contains cosmetic components that improve consumer properties, namely: aromatic substances, and/or pigments, and/or preservatives.

12. The mask according to claim 1, **characterized in that**, as an electrolyte substance containing multiply charged ions, it contains divalent metal salts, for example, calcium chloride, or zinc chloride, or zinc sulfate, or magnesium sulfate; or polybasic organic and inorganic acids and/or their salts, for example, boric acid, or citric acid, or sodium citrate.

13. The mask according to claim 1, **characterized in that** the additional reagent is an aqueous solution of an electrolyte substance, which contains distilled or double distilled or flower water as water.

14. The mask according to claim 13, **characterized in that**, as the flower water, the aqueous solution of the electrolyte substance contains lavender flower water, or melissa flower water, or pink flower water.

15. A method of applying a cosmetic mask to a skin, comprising:
applying a creamy cosmetic product with a fat, emulsion, or gel base onto the skin and then applying an additional reagent on top of it, **characterized in that** the creamy cosmetic product is first applied to the skin, containing an additional water-soluble biocompatible polymer with a mass in the range of 50,000 - 1,000,000 Da, capable of undergoing a cross-polymerization reaction, and selected from the group including: polyvinyl alcohol, polyvinylpyrrolidone, water-soluble polysaccharide modifications, water-soluble polysaccharide copolymers and mixtures thereof;
incubating for 5-7 minutes, and then
applying the additional reagent on top of the creamy cosmetic product, wherein the additional reagent is a 0.5-15.0% aqueous solution of an electrolyte substance containing multiply charged ions, while weight ratio of the water-soluble biocompatible polymer and the electrolyte substance in the aqueous solution is from 10:1 to 1:1, respectively.

16. The method according to claim 15, **characterized in that** the aqueous solution of the electrolyte substance is applied in the form of a spray or aerosol sprayed onto a surface of the applied creamy cosmetic product for no more than 2-3 seconds.
